# EUROPEAN PATENT SPECIFICATION

(11) **EP 2 328 512 B1**
(45) Date of publication and mention of the grant of the patent: **06.04.2016**
(21) Application number: 09811944.9
(22) Date of filing: 18.08.2009
(51) Int. Cl.: A61F 2/06

(54) **Stent graft having extended landing area**
Stentprothese mit erweiterter Landezone
Greffe de stent comportant une surface de mise en place étendue

(30) Priority: 25.08.2008 US 197604
(43) Date of publication of application: 08.06.2011
(73) Proprietor: St. Jude Medical, Cardiology Division, Inc., St. Paul MN 55117 (US)
(72) Inventor: REN, Brooke, Maple Grove MN 55311 (US)
(74) Representative: Potter Clarkson LLP
(86) International application number: PCT/US2009/054155
(87) International publication number: WO 2010/027651

(56) References cited:
- EP-A1- 1 913 897
- US-A1- 2004 039 438
- US-A1- 2004 249 447
- US-A1- 2006 161 241
- US-A1- 2006 161 241
- US-A1- 2007 005 127

## Description

### FIELD OF THE INVENTION

The present invention relates to medical devices and associated methods for treating various target sites and, in particular, to medical devices configured for use in arcuate lumens and associated methods for delivering such medical devices.

### BACKGROUND OF THE INVENTION

An aortic aneurysm is a weak area in the aorta wall, which may be caused, for example, by arteriosclerosis. As blood flows through the aorta, the weak area of the vessel wall thins over time and expands like a balloon. Most commonly, aortic aneurysms occur in the portion of the vessel below the renal artery origins.

Eventually, an untreated aortic aneurysm will burst if the vessel wall gets too thin. Such rupturing of an aortic aneurysm frequently leads to death. As such, once an aneurysm reaches about 5 cm in diameter, it is usually considered necessary to treat to prevent rupture (below 5 cm, the risk of the aneurysm rupturing is considered lower than the risk of conventional heart surgery in patients with normal surgical risks).

Aneurysms, including aortic aneurysms, may be treated with surgery. The surgical procedure for treating an aortic aneurysm involves replacing the affected portion of the aorta with a synthetic graft, usually comprising a tube made out of an elastic material with properties very similar to that of a normal, healthy aorta. However, surgical treatment is complex and may pose additional risks to the patient, especially the elderly.

More recently, instead of performing surgery to repair an aortic aneurysm, vascular surgeons have installed an endovascular stent graft delivered to the site of the aneurysm using elongated catheters. An endovascular stent graft is a tube composed of blood impervious fabric supported by a metal mesh called a stent. It can be used for a variety of conditions involving the blood vessels, but most commonly is used to reinforce aneurysms. Typically, the surgeon will make a small incision in the patient's groin area and then insert into the vasculature a delivery catheter containing a collapsed, self-expanding or balloon-expandable stent graft. The delivery catheter is advanced to a location bridging the aneurysm, at which point the stent graft is delivered out from the delivery catheter and expanded to approximately the normal diameter of the aorta at that location. Over time, the stent graft becomes endothelialized and the space between the outer wall of the stent graft and the aneurysm ultimately fills with clotted blood, which prevents the aneurysm from growing further.

Depending on where the location of the aneurysm is within a vessel relative to other branch vessels, different design variations of the stent graft may be needed. For example, in treating an aortic aneurysm in the area of the renal arteries, the stent graft should be placed so as not to exclude blood flow through the renal arteries. Moreover, the stent graft should be anchored within the lumen, such as by promoting endothelialization or fixation with the lumen, in order to reduce the incidence of migration. Enhanced fixation of the stent graft to the arterial wall may also reduce the occurrence of endoleaks or blood flowing around the stent, which may prevent further weakening of the arterial wall at the site of the aneurysm.

Providing for adequate fixation of a stent graft in the area of the aortic arch can be challenging due to the various arteries that branch from the aorta in that region. The stent graft must provide adequate contact force against the vessel walls to prevent migration and endoleaks, but must not restrict blood flow to the branching arteries.

Therefore, there is a need for a stent graft that is capable of being deployed in a lumen having an arcuate portion, such as in the vicinity of the aortic arch. The stent graft should easily be deliverable and should be capable of being adequately anchored within the lumen.

US 2006/0161241 describes a radially expanding stent for use in the aortic arch comprising a generally cylindrical member having a proximal opening, a distal opening and a lumen therebetween, and a side opening in the wall of the generally cylindrical member. The side opening allows blood flow to pass to a number of branching vessels. The stent may be self-expanding and formed of a superelastic material such as Nitinol. EP 1913897 describes a self-expandable stent and synthetic vascular graft for use in the aorta. A window divides the stent / graft into two separate parts connected by alloy wire. US 2007/0005127 describes a device having a myocardial section configured to be positioned in a heart wall between a coronary vessel and a chamber of the heart and a vessel section configured to be positioned in the coronary vessel. The vessel section is connected to the myocardial section and configured to articulate relative thereto.

### SUMMARY OF THE INVENTION

The present invention provides a medical device according to claim 1. More generally described herein is a medical device, such as, for example, a stent graft, for treating a target site within the body. For example, one embodiment provides a medical device for treating a target site within a lumen having an arcuate portion and at least one branch lumen extending therefrom. The medical device includes a first tubular portion comprising a proximal and distal end, and a second tubular portion comprising a proximal and distal end. The first and second tubular portions each may include at least one layer of a metallic material, such as a shape memory alloy, that is configured to be heat set to an expanded heat set configuration, and in some cases may include multiple layers. The first and second tubular portions may each be configured to be constrained to a smaller diameter than the respective expanded heat set configuration, for example, for delivery within a catheter, and may return to the respective expanded heat set configuration when deployed from the catheter.

The medical device further includes a linking portion that is a braid, that couples the first and second tubular portions. At least part of the linking portion has a preset, memorized arcuate configuration that is configured to conform to at least a portion of the arcuate portion of the lumen. The linking portion may be resilient and/or adjustable in length. An opening is defined between the distal end of the first tubular portion and the proximal end of the second tubular portion and is configured to align with the at least one branch lumen and facilitate fluid flow between the at least one branch lumen and the arcuate portion of the lumen. According to one aspect, a location of each of the linking portion and the opening within the arcuate portion of the lumen may be rotationally dependent on a location of the at least one branch lumen.

The first tubular portion may be, for example, a stent graft configured to be positioned downstream of the arcuate portion of the lumen, such as within a descending thoracic aorta. The second tubular portion may be configured to anchor the medical device upstream of the arcuate portion of the lumen, such as within an ascending thoracic aorta. The linking portion is configured to be positioned within the arcuate portion of the lumen, such as within an aortic arch, such that the opening defined between the first and second tubular portions is configured to align with at least one branch lumen extending from the arcuate portion of the lumen. The first tubular portion, said second tubular portion, and linking portion comprise a single braided metallic tube.

In another embodiment, a medical device for treating an aneurysm within an aortic arch is provided. The medical device includes a first tubular portion configured to be positioned within a descending thoracic aorta and a second tubular portion configured to be positioned within an ascending thoracic aorta. The medical device further includes a linking portion coupling the first and second tubular portions and an opening defined between the first and second tubular portions. The linking portion is configured to be positioned within, and conform at least partially to, the aortic arch. The opening is configured to align with at least one artery extending from the aortic arch.

A method of delivering a medical device to a target site within a lumen having an arcuate portion and at least one branch lumen extending therefrom is provided. The method includes providing a medical device that has a first tubular portion comprising a proximal and distal end, a second tubular portion comprising a proximal and distal end, and a linking portion coupling the first and second tubular portions. The medical device also includes an opening defined between the distal end of the first tubular portion and the proximal end of the second tubular portion. The first and second tubular portions and the linking portion can be constrained from respective expanded configurations to a smaller diameter for delivery within a catheter, for example, by respectively axially elongating the tubular and linking portions. The medical device can be delivered, for example, over a guidewire, to the target site, where the device can be deployed from the catheter such that the first and second tubular portions respectively assume their expanded configurations, the linking portion conforms to the arcuate portion of the lumen, and the opening aligns with the at least one branch lumen and facilitates fluid flow between the at least one branch lumen and the arcuate portion of the lumen.

The first and second tubular portions respectively self-expand and return to their expanded configurations when deployed from the catheter. In other embodiments, the first and second tubular portions may be axially compressed so as to urge the first and second tubular portions to return to the respective expanded configurations. In some embodiments, the medical device can be deployed from the catheter such that the second tubular portion is disposed within the ascending thoracic aorta of the body, the first tubular portion is disposed within the descending thoracic aorta of the body, and the linking portion is disposed within, and conforms generally to, at least a portion of the shape of the aortic arch. In addition, the deployment of the medical device may be rotationally dependent on a location of each of the linking portion and the opening within the arcuate portion of the lumen with respect to a location of the at least one branch lumen.

### BRIEF DESCRIPTION OF THE DRAWINGS

Having thus described the invention in general terms, reference will now be made to the accompanying drawings, which are not necessarily drawn to scale, and wherein:
Fig. 1 is a perspective view of a medical device configured in accordance with an exemplary embodiment;
Fig. 2 is a perspective view of the medical device of Fig. 1 in a constrained configuration;
Fig. 3 is a cross-sectional view of the medical device of Fig. 1 deployed at a target site around an aortic arch;
Figs. 4-6 are perspective views demonstrating a process for fabricating a medical device in accordance with an exemplary embodiment;
Fig. 7 is a side view of the medical device of Fig. 1 disposed within the bore of a delivery catheter;
Fig. 8 is a perspective view of a medical device configured in accordance with another exemplary embodiment;
Fig. 9 is a cross-sectional view of the medical device of Fig. 8 taken along line 9-9 of Fig. 8;
Figs. 10 and 11 are perspective views of medical devices configured in accordance with still other exemplary embodiments;
Fig. 12 is an exploded perspective view of the medical device of Fig. 10;
Figs. 13A-B are perspective views demonstrating a process for fabricating a medical device according to an additional embodiment;
Fig. 14 is a perspective view of a medical device according to another embodiment of the present invention; and
Figs. 15-17 are perspective views of medical devices having an adjustable linking portion.

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth wherein, rather, these embodiments are provided so that this disclosure will be thorough and complete, and will fully convey the scope of the invention to those skilled in the art. Like numbers refer to like elements throughout.

Embodiments of the present invention provide a medical device for use in treating a target site within the body, such as excluding or occluding various vascular abnormalities, which may include, for example, excluding an aneurysm. The device may also be used as a graft for lining a lumen of a vessel. It is understood that the use of the term "target site" is not meant to be limiting, as the device may be configured to treat any target site, such as an abnormality, a vessel, an organ, an opening, a chamber, a channel, a hole, a cavity, or the like, located anywhere in the body. For example, the abnormality could be any abnormality that affects the shape or the function of the native lumen, such as an aneurysm, a lesion, a vessel dissection, flow abnormality or a tumor. Furthermore, the term "lumen" is also not meant to be limiting, as the abnormality may reside in a variety of locations within the vasculature, such as a vessel, an artery, a vein, a passageway, an organ, a cavity, or the like.

As used herein the term "proximal" shall mean closest to the operator (less into the body) and "distal" shall mean furthest from the operator (further into the body). In positioning of the medical device from a downstream access point, distal is more upstream and proximal is more downstream.

As explained in further detail below, embodiments of the present invention provide medical devices for treating various target sites. The medical devices may include tubular portions separated and coupled by an arcuate or flexible linking portion. The arcuate or flexible linking portion may allow the medical device to conform to an arcuate target site. Moreover, the arcuate or flexible linking portion may enable the tubular portions to be disposed on opposing sides of an arcuate portion of a target site, which may improve the fixation of the medical device at such an arcuate target site. Furthermore, the medical device may include an opening configured to align with one or more branch lumens extending from the arcuate portion in order to reduce blockage of the one or more branch lumens.

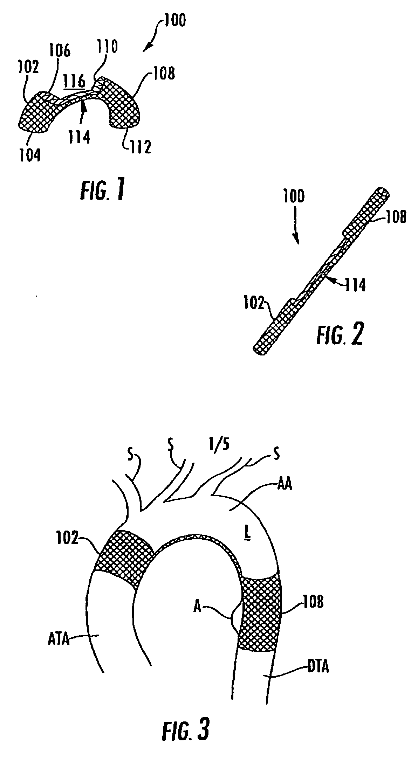

Referring to Fig. 1, therein is shown a medical device 100 configured in accordance with an exemplary embodiment. The medical device 100 includes a first tubular portion, such as a stent graft 108, which has a proximal 112 end and a distal end 110. The medical device 100 also includes a second tubular portion, such as an anchoring structure 102, having a proximal end 106 and a distal end 104. Each of the first and second tubular portions may be generally cylindrical, but could be various shapes depending on the configuration of the lumen in which the tubular portions are to be positioned. An arcuate/linking portion 114 couples the stent graft 108 and anchoring structure 102, and an opening 116 extending between the proximal end 106 of the anchoring structure 102 and the distal end 110 of the stent graft 108.

Referring to Figs. 1 and 2, one or both of the stent graft 108 and anchoring structure 102 may include at least one layer of a metallic material, the layer being in the form of a braided metallic tube. The fabric can be composed of multiple metallic strands. Although the term "strand" is discussed herein, "strand" is not meant to be limiting, as it is understood the fabric may comprise one or more wires, cords, fibers, yams, filaments, cables, threads, or the like, such that such terms may be used interchangeably. The stent graft 108 and anchoring structure 102 may be a variety of occlusive materials capable of at least partially inhibiting blood flow therethrough in order to facilitate the formation of thrombus and epithelialization around the device. Moreover, the stent graft 108 and anchoring structure 102 are a self-expandable material, such as stainless steel or etched stents.

In some embodiments, one or both of the stent graft 108 and anchoring structure 102 may include multiple layers of metallic material. The layers may have different porosity or opening sizes. In addition, one layer may be for structural support and a second layer may inhibit blood flow through the layer. The layer(s) of a metallic material are configured to be heat set to an expanded heat set configuration. For example, in one embodiment, one or both of the stent graft **108** and anchoring structure **102** can be composed at least partially of a shape memory material in order to provide for being heat set in an expanded configuration and to retain the expanded shape at or below body temperature. The stent graft **108** and anchoring structure **102** can then be configured to be constrained to a smaller diameter than their respective expanded heat set configurations for delivery through a catheter to a target location within the body. For example, the stent graft **108** and anchoring structure **102** may be braided tubular structures that have expanded configurations in which the outer diameters of the stent graft and anchoring structure are approximately the same as the inner diameter of the aorta, and which can be reduced to smaller diameters for delivery within a catheter, such as by axially elongating the stent graft and anchoring structure. The linking portion may be configured to pass through a catheter without necessarily needing any axial elongation.

In one embodiment, the stent graft **108,** anchoring structure **102,** and/or linking portion **114** are formed from a shape memory alloy, such as Nitinol. It is also understood that the stent graft **108,** anchoring structure **102,** and/or linking portion **114** may comprise various materials other than Nitinol that have highly elastic properties, such as spring stainless steel, and alloys such as Elgiloy®, Hastelloy®, CoCrNi alloys (e.g., trade name Phynox), MP35N®, or CoCrMo alloys.

According to one embodiment, each layer of the device may comprise 36-144 wire strands ranging in diameter from about 0.0005 to 0.010 in. formed of a shape memory alloy that are braided so as to define fenestrations with an area of about 0.00015 to 0.0015 sq. in., which are sufficiently small so as to slow the blood flow through the wall of the device and to facilitate thrombus formation thereon. Inner and outer braided layers may have pitch angles that are about equal to obtain desirable collapse and expansion characteristics, such as maintaining a uniform overall length. The stiffness of the device may be increased or decreased by altering the wire strand size, the shield angle, the pick rate, and the number of wire strand carriers or the heat treatment process.

Thus, the stent graft **108** can also be configured to facilitate thrombosis, for example, by at least partially inhibiting blood flow therethrough in order to facilitate the formation of thrombus and epithelialization around the stent graft. In particular, the braid of a metallic fabric may be chosen to have a predetermined pick and pitch to define openings or fenestrations so as to vary the impedance of blood flow therethrough. For instance, the formation of thrombus may result from substantially precluding or impeding flow, or functionally, that blood flow may occur for a short time, e.g., about 3-60 minutes through the metallic fabric, but that the body's clotting mechanism or protein or other body deposits on the braided wire strands results in occlusion or flow stoppage after this initial time period. For instance, occlusion may be clinically represented by injecting a contrast media into the upstream lumen of the stent graft **108** and if no contrast media flows through the wall of the stent graft after a predetermined period of time as viewed by fluoroscopy, then the position and occlusion of the stent graft is adequate. Moreover, occlusion of the target site could be assessed using various ultrasound echo doppler modalities. Although the stent graft **108** has been described as having one or more layers of occlusive material, it is understood that the anchoring structure **102** and/or linking portion **114** may also or alternatively include one or more layers of occlusive material to facilitate thrombosis or the wires may be coated with a thrombus promoting substance.

According to one embodiment, the stent graft **108** could be configured to be positioned within a lumen having an aneurysm. For instance, the stent graft **108** could be positioned within a lumen having an aneurysm *A* in the descending thoracic aorta (*DTA*). In addition or alternatively, the anchoring structure **102** could comprise occlusive material and be configured to exclude an aneurysm in the ascending thoracic aorta *(ATA).*

The linking portion **114** has a preset, memorized arcuate configuration. In some embodiments, the arcuate configuration may conform to at least a portion of an arcuate portion of the lumen of a vessel, this aspect making such embodiments well-suited for deployment within a lumen having an arcuate portion, such as, for example, the aortic arch (*AA*). For example, referring to Fig. 3, the stent graft **108** can be configured to be positioned downstream of the arcuate portion of the lumen *L* (*e.g.,* in the *DTA*), the anchoring structure **102** can be configured to anchor the medical device upstream of the arcuate portion of the lumen (e.g., in the ATA), and the linking portion 114 can be configured to be positioned within the arcuate portion of the lumen (e.g, in the AA). In this way, the opening 116 defined between the stent graft 102 and the anchoring structure 108 may be configured to align with at least one side or branch lumen S extending from the arcuate portion of the lumen L. Thus, the linking portion 114 may be configured to conform to the arcuate portion of the lumen opposite the branch lumens S, while the opening 116 is configured to facilitate fluid flow between the arcuate portion and the branch lumens. Thus, the location of the linking portion 114 and the opening 116 may be rotationally dependent on one another.

The linking portion 114 includes a braid. The linking portion 114 can be composed at least partially of a shape memory material and is heat set in the arcuate configuration.

The curvature of the linking portion 114 and/or orientation of the stent graft 108 to the anchoring structure 102 with respect to one another may vary depending on the particular arcuate lumen being treated or a particular patient. In many cases, the linking portion 114 may be resilient, either due to the material used to form the linking portion, the geometry of the linking portion, or both. For example, the reduced cross-section of the linking portion 114 (e.g., relative to that of the stent graft and anchoring structure 108, 102) may make an otherwise straight linking portion sufficiently flexible to conform to an arcuate portion of a lumen.

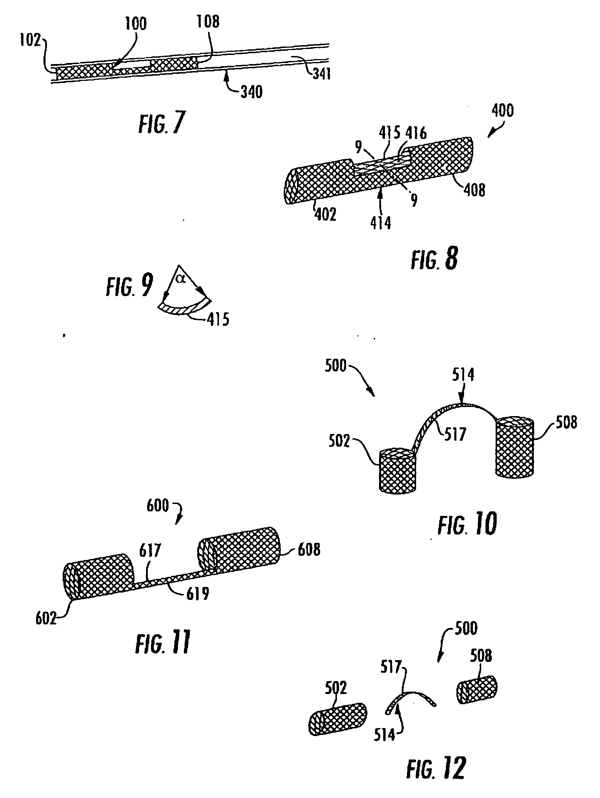

The size and configuration of the opening 116 may depend on the particular linking portion 114 employed. In addition, the size and configuration of the opening 116 chosen may depend on the number and location of branch lumens to be aligned with the opening. For example, a linking portion 114 comprising a thin or small diameter wire or band would provide a large opening 116 (see e.g., FIG. 10), whereas an opening defined in a braided fabric may be much smaller (see e.g., FIGS. 1 and 8). Furthermore, in one embodiment, the linking portion **114** may include a "loose" fabric that is less densely braided than the stent graft **108** and anchoring structure **102,** such that the blood may readily How through the larger openings **116** defined in the loose fabric. Therefore, at least one opening **116** may be defined in the linking portion **114** and may be located at one or more locations in the linking portion (including up to about the entire circumference of the linking portion).

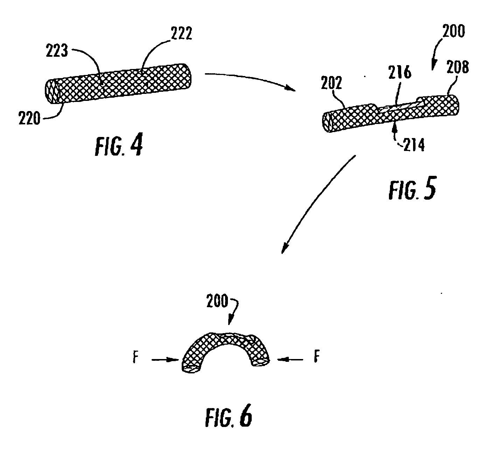

Still referring to Figs. 1 and 2, the stent graft **108,** anchoring structure **102,** and linking portion **114** are integrally formed from a common material. For example, referring now to Figs. 4-6, a single braided metallic (e.g., Nitinol) tube **220** can be formed by partially cross cutting the tube **220** along sections **222, 223** (e.g., by cutting the wire strands) in order to form a medical device **200** having a linking portion **214** and opening **216** between a first tubular portion **208** and a second tubular portion **202.** The linking portion **214** is formed by axial elongation to reduce its diameter and constraining the linking portion during a heat setting operation to memorize the constrained diameter. During the same heat treatment process, the stent graft **108** and anchoring structure **102** may be heat set in their expanded diameters to memorize their shape. The braided tube **220** may also be heat formed so as to have an arcuate region in the portion that will become the linking portion **214,** or can be processed after forming the linking portion **214** such that the linking portion assumes an arcuate configuration, for example, by being forced (e.g., via forces **F** in Fig. 6) into an arcuate shape and then heat set.

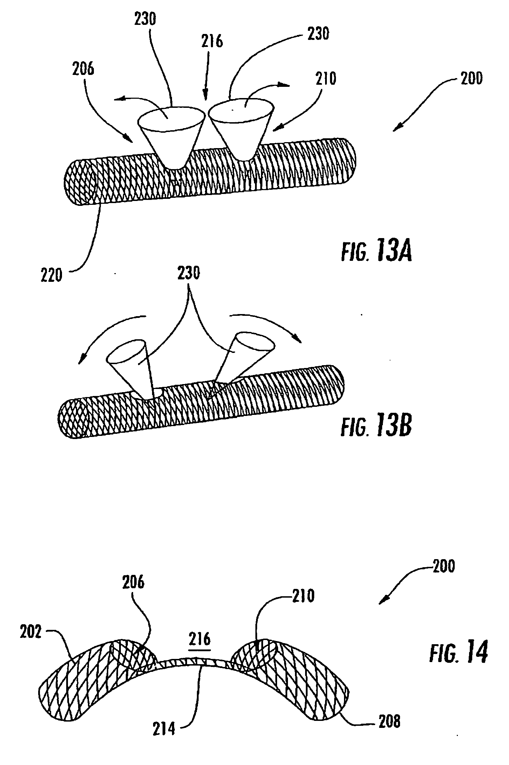

Figs. 13A-B and 14 illustrate a medical device **200** according to another embodiment of the present invention. The device **200** may be formed from a single length of tubular braided shape memory alloy capable of being heat treated to have a shape transformation temperature below body temperature (e.g., 20-37° C). Openings **216** can be formed in the tube **220** by pushing a cone shaped probe **230** into the side wall at one location but typically two axial aligned locations along the outer surface to form the openings **206, 210** as shown in Fig. 13B. Once the wires have been displaced by the conical probes **230** sufficiently to establish the desired opening diameter, the braided portion between the two openings **206,210** can be axially elongated to form the linking portion **214** and opening **216.** The process of forming the device **200** will result in loose wires that need to be manually realigned by axial tension from either wire ends while holding the device in the desired final device shape. Once the wires are aligned as desired, the device may be heat set to memorize the desired final shape. The final device shape as shown in Fig. 14 has formed openings **206, 210** at an angle less than perpendicular to the central axis of the tubular portions **202, 208.**

Referring to Figs. 8 and 9, therein is shown a medical device **400** with the stent graft **408** and anchoring structure **402** disposed coaxially with one another according to one embodiment of the present invention. The opening **416** extends between the stent graft **402** and anchoring structure **408** and may be defined, in cross section, by a curved (e.g., circular) sector having an angle a between 0 and 360 degrees. For example, the opening **416** may have an angle a in the range of about 45 to about 225 degrees. The medical device **400** shown in Figs. 8 and 9 could be resilient structure and thereby conform to an arcuate vessel or heat set in the arcuate configuration as described above.

Referring to Figs. 10-12, therein are shown medical devices **500, 600** configured in accordance with further exemplary embodiments. The devices **500, 600** include first tubular portions **508, 608** and second tubular portions **502, 602.** A linking portion **517, 617** couples the corresponding first and second tubular portions **508, 502, 608, 602.** As indicated above, the linking portion **517, 617** includes a braid In either case, the thickness of the linking portion **517, 617** can be sufficiently small as compared to its length so as to be relatively flexible. The flexibility of the linking portion **517, 617** may, in some cases, facilitate either delivery of the device **500, 600** to a target site in the body or the ability of the device to conform to the target site once delivered.

In a further examples the medical device **600** may be fabricated by laser cutting or acid etching a pattern into a shape memory tube to form the first and second tubular portions **602, 608** (see Fig. 11) by removing most of the circumference of a central portion between the tubular portions to leave a remainder linking portion **614** and corresponding opening **616**. Alternatively, the tubular portions **602, 608** may be fabricated as individual components and connected to a separate linking portion 617 similar to that shown in Fig. 12. The tubular portions **602, 608** may be manually expanded to the desired diameter and/or curved to an arcuate preset shape and along with the linking portion **617,** heat set in an oven while constrained to the desired final shape to memorize the desired final device shape. The tubular portions **602, 608** may be radially compressed in diameter or elongated for delivery through a catheter to a treatment site within the body. The device may self expand to the memorized shape upon exiting the catheter. Catheter based delivery devices for self expanding stents may be an appropriate means for delivery of the medical device **600.** It should be noted that the devices **100, 200, 400, 500, 600** may be sized larger than the vessel diameter by 10-30% to ensure that the device exhibits an anchoring force against the vessel wall. The devices, therefore, may not achieve 100% of their preset shape when exiting a catheter restraint due to vessel resistance to expansion.

Referring again to Fig. 1, in some embodiments, the linking portion **114** may be adjustable in length. For example, the linking portion **114** may include a compressed braid that can be selectively decompressed (and, in some cases, re-compressed) to an extent that is adjustable. Alternatively, the linking portion **114** can include a series of everting links.

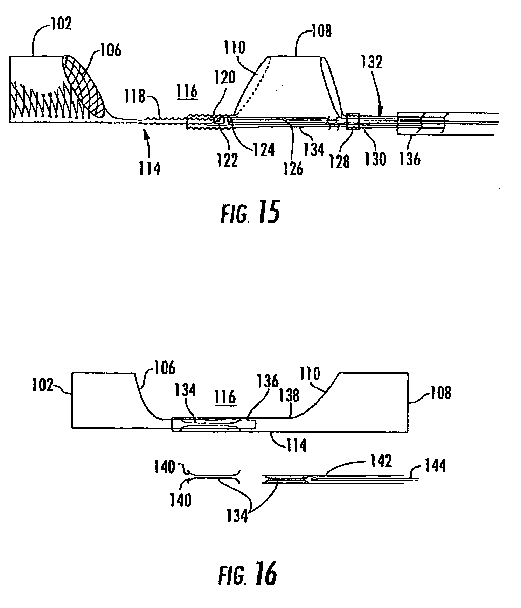

Fig. 15 illustrates one example for facilitating the length adjustment of the linking portion **114.** In particular, Fig. 15 illustrates that the anchoring structure **102** and stent graft **108** may include respective threaded portions **118, 120** configured to engage one another. The anchoring structure **102** may include a threaded connector **122** that is configured to engage a threaded end **124** on a distal delivery device **126.** The stent graft **108** may also include a threaded connector **128** that is configured to engage a threaded end **130** on a proximal delivery device **132**. The distal delivery device **126** is deliverable through an internal sheath **134** and catheter **136.** Thus, both the distal delivery device **126** and internal sheath **134** are configured to be axially displaced through the threaded connector **128** and proximal delivery device **132.** The length of the linking portion **114** may be adjusted by threading the threaded portions **118**, **120** with respect to one another, which may occur before delivery of the device based on an image of the target site (e.g., using fluoroscopy), or the device could be removed prior to being fully deployed and the length of the linking portion adjusted. When the threaded ends **124, 130** are engaged with respective threaded connectors **122**, **128**, rotation of the distal **126** and proximal **132** delivery devices results in adjustment of the length of the linking portion **114** as the threaded portions **118,120** are rotated with respect to one another.

Fig. 16 illustrates another example wherein the length of the linking portion **114** may be adjusted using a locking member 134. More specifically, the locking member **134** may be configured to engage respective free ends **136, 138** of the anchoring structure **102** and stent graft **108.** Thus, once the free ends **136, 138** have been axially displaced with respect to one another to achieve a desired length of the linking portion **114,** the locking member **134** may engage the free ends together to prevent any further axial displacement. The locking member **134** may include a pair of hook-shaped members **140** that are configured to engage the free ends **136**, **138** and may include various materials, such as a metallic material. The locking member **134** may be configured to self expand upon release from a delivery catheter **142,** which may be facilitated by a pusher shaft **144,** wherein both the delivery catheter and pusher shaft are capable of being axially displaced within the free ends **136,138** to a desired location prior to release of the locking member. The end of the delivery catheter **142** may include a material capable of resisting puncture by the locking member **134** and facilitate axial displacement of the locking member out of the delivery catheter. For example, the distal end of the delivery catheter **142** may be a metallic material or reinforced sleeve, while the remaining portion of the delivery device may be a flexible, polymeric material. The locking member **134** may be radially constrained for delivery within the delivery catheter **142,** and the pusher shaft **144** may be used to push the locking member out of the delivery catheter.

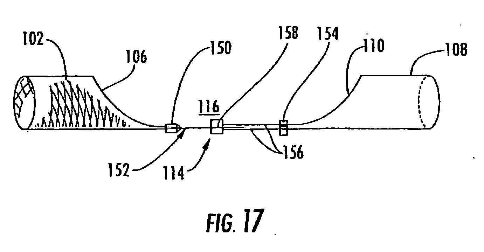

Another example of a medical device having an adjustable linking portion **114** is illustrated in Fig. 17. In this particular embodiment, the anchoring structure 102 includes a clamp **150** and a single wire **152** extending proximally therefrom. Similarly, the stent graft **108** includes a clamp **154** and a pair of wires **156** extending distally therefrom, wherein the pair of wires are configured to extend over the wire **152.** Once a desired length of the linking portion **114** is obtained by axially displacing the wire **152** and the pair of wires 156 with respect to one another, the wires 152, 156 may be crimped together with a clamp 158 or using any other suitable techniques for securing the wires together, such as with a set screw.

Referring to Figs. 1-3 and 7, in order to deliver the medical device 100 to a target site within a lumen having an arcuate portion, such as an aortic arch, the stent graft 108 and the anchoring structure 102 can be constrained from respective expanded configurations (shown in Fig. 1) to a smaller diameter (shown in Fig. 2). For example, where the stent graft 108 and the anchoring structure 102 are formed of a braided metallic fabric, each of the stent graft and the anchoring structure may have a first diameter and may be capable of being collapsed to a second, smaller diameter by being axially elongated.

The constrained device 100 can then be positioned in a delivery catheter 340, which is a catheter that defines an axial bore 341. In this way, the device 100 is maintained in the constrained configuration during delivery by the wall defining the bore 341 of the catheter 340. The catheter 340 and device 100 can then be advanced, for example, over a guidewire, until disposed at the target site (in this case the aortic arch area), where the device 100 can be deployed from the catheter. Once the device 100 has been deployed completely out of the catheter 340, the stent graft 108 and anchoring structure 102 may assume the expanded shape (to the extent permitted by the surrounding vasculature, e.g., the ascending and descending thoracic aorta, respectively) and the linking portion may conform to the arcuate portion of the lumen (e.g., the aortic arch). Further examples of the procedures by which a medical device configured in accordance with exemplary embodiments can be delivered are provided in U.S. Patent Appl. Publ. No. 2006/0253184 filed May 4, 2005.

In some embodiments, the stent graft 108 and anchoring structure 102 may self-expand upon being deployed from the catheter 340 as the constraining forces of the catheter are removed. In other examples, the stent graft 108 and anchoring structure 102 may be physically urged into or toward the expanded shape, say, by inflating a balloon located within the stent graft and anchoring structure, or by axially compressing the tube following deployment from the catheter 340.

The location of the medical device **100** may be rotationally dependent on the location of one or more branch lumens extending from the arcuate lumen, such as the *AA.* Thus, the linking portion **114** may be positioned opposite the openings of the branch lumens, while the opening **116** may be configured to align with the openings of the branch lumens in order to facilitate fluid flow therethrough. In order to aid in the alignment of the medical device within the lumen, the medical device may also comprise one or more radiopaque markers to indicate angular orientation of the device such that the linking portion **114** is located along the inside smallest radius of the arcuate lumen (see e.g., Fig. 3). For example, radiopaque markers could line the linking portion or the openings of the tubular portions adjacent the ostia of the branch lumens, and/or the braid itself could include one or more radiopaque strands so that the medical device is properly positioned and does not block any branch lumens. Radiopaque markers may also facilitate location of the anchoring portion **102** and the stent graft **108** relative to desired target locations. It is further contemplated that the expanded diameter portions of the anchoring portion 102 and the stent graft 108 may be heat set to incorporate a corrugated portion or a sinusoidal wave pattern in the outer surface to increase radial strength as described in pending U.S. Appl. No. 12/181,639, entitled Medical Device including Corrugated Braid and Associated Method published as US2010/0030321 A1. The anchoring portion **102** and/or the stent graft **108** may additionally comprise hooks for engaging the lumen to ensure the device does not migrate.

Embodiments of the present invention may provide several advantages. For example, the medical device is capable of conforming to a variety of arcuate portions within a vessel and is, thus, adaptable for a variety of target sites and patients. The medical device may include a heat set or resilient linking portion that facilitates such adaptability. The linking portion may include an opening that is configured to align with one or more branch vessels extending from the arcuate portion such that the opening reduces blockage in the arcuate portion, such as in the aortic arch. The medical device may also include a stent graft configured to facilitate occlusion at a target site, such as at an aneurysm. Moreover, the medical device may include an anchoring structure in order to facilitate fixation within the vessel and reduce the incidence of migration. Therefore, the medical device is capable of treating target sites within a vessel that may be otherwise difficult to anchor therein or susceptible to blockage of- branch vessels when a conventional stent graft is employed.

Many modifications and other embodiments of the invention will come to mind to one skilled in the art to which this invention pertains having the benefit of the teachings presented in the foregoing descriptions and the associated drawings. Other embodiments are within the scope of the appended claims.

## Claims

1. A medical device (100) for treating a target site within a lumen having an arcuate portion (L) and at least one branch lumen (S) extending therefrom, said medical device comprising:
a first tubular portion (108) comprising a proximal end (112) and a distal end (110);
a second tubular portion (102) comprising a proximal end (106) and a distal end (104), the first and second tubular portions each comprising at least one layer of metallic shape memory alloy material, being heat set to an expanded heat set configuration;
a resilient linking portion (114) coupling said distal end of the first tubular portion and the proximal end of the second tubular portion and comprising a preset, memorized arcuate configuration configured to conform to at least a portion of the arcuate portion (L) of the lumen opposite the at least one branch lumen (S), the linking portion configured to assume its preset, memorized arcuate configuration upon being deployed at the target site, the linking portion comprising a metallic shape memory alloy and having been constrained during a heat setting operation so as to be heat set in an axially elongated configuration to reduce its diameter and to memorize the axially elongated, reduced diameter configuration; and
an opening (116) defined between the distal end (110) of said first tubular portion (108) and the proximal end (106) of said second tubular portion (102), wherein said opening is configured to align with the at least one branch lumen (S) and facilitate fluid flow between the at least one branch lumen and the arcuate portion (L) of the lumen;
wherein said first tubular portion, said second tubular portion, and said linking portion comprise a single braided metallic tube.

2. The medical device of Claim 1, wherein said at least one layer of metallic material comprises a plurality of layers of metallic material.

3. The medical device of Claim 1, wherein said first and second tubular portions (108, 102) are each configured to be constrained to a smaller diameter than the respective expanded heat set configuration for delivery within a catheter and return to the respective expanded heat set configuration when deployed from the catheter.

4. The medical device of Claim 1, wherein said linking portion (114) is adjustable in length.

5. The medical device of Claim 1, wherein said first tubular portion (108) is a stent graft configured to be positioned downstream of the arcuate portion (L) of the lumen.

6. The medical device of Claim 5, wherein said second tubular portion (102) is configured to anchor the medical device upstream of the arcuate portion of the lumen (L).

7. The medical device of Claim 1, wherein a location of each of said linking portion (114) and said opening (116) within the arcuate portion (L) of the lumen is rotationally dependent on a location of the at least one branch lumen (S).

8. The medical device of Claim 1, wherein said first tubular portion (108) is configured to be positioned within a descending thoracic aorta, said second tubular portion is configured to be positioned within an ascending thoracic aorta, and said linking portion (114) is configured to be positioned within an aortic arch, and wherein said opening (116) is configured to align with at least one artery extending from the aortic arch.

9. The medical device of Claim 1, wherein said first and second tubular members (108, 102) are self-expandable.

10. The medical device of Claim 1, wherein said first and second tubular portions (108, 102) are curved to an arcuate preset shape.

11. The medical device of Claim 1, wherein said target site within the lumen includes an aortic aneurysm.

12. The medical device of Claim 1, wherein at least one layer is configured to at least partially inhibit blood flow therethrough.

13. The medical device of Claim 1, wherein the linking portion has a diameter smaller than a diameter of each of the first and second tubular portions when the first and second tubular portions are in the expanded heat set configuration.

14. The medical device of Claim 1, wherein the cross section of the linking portion is less than the expanded cross sections of the first and second tubular portions.

## Patentansprüche

1. Medizinische Vorrichtung (100) zum Behandeln einer Zielstelle in einem Lumen mit einem bogenförmigen Teil (L) und wenigstens einem sich davon erstreckenden Zweiglumen (S), wobei die medizinische Vorrichtung Folgendes umfasst:
einen ersten röhrenförmigen Teil (108), umfassend ein proximales Ende (112) und ein distales Ende (110);
einen zweiten röhrenförmigen Teil (102), umfassend ein proximales Ende (106) und ein distales Ende (104), wobei der erste und der zweite röhrenförmige Teil jeweils wenigstens eine Schicht aus metallischem Formgedächtnislegierungsmaterial umfassen, die auf eine erweiterte thermofixierte Konfiguration thermofixiert ist;
einen elastischen Verbindungsteil (114), der das distale Ende des ersten röhrenförmigen Teils und das proximale Ende des zweiten röhrenförmigen Teils verbindet und eine voreingestellte, eingeprägte bogenförmige Konfiguration umfasst, die konfiguriert ist, mit wenigstens einem Teil des bogenförmigen Teils (L) des Lumens gegenüber dem wenigstens einen Zweiglumen (S) übereinzustimmen, wobei der Verbindungsteil konfiguriert ist, seine voreingestellte, eingeprägte bogenförmige Konfiguration einzunehmen, nachdem er an der Zielstelle eingesetzt worden ist, wobei der Verbindungsteil eine metallische Formgedächtnislegierung umfasst und während eines Thermofixierungsvorgangs beschränkt war, um in einer axial verlängerten Konfigurationen thermofixiert zu werden, um seinen Durchmesser zu reduzieren und die axial verlängerte, reduzierte Durchmesserkonfiguration einzuprägen; und
eine Öffnung (116), die zwischen dem distalen Ende (110) des ersten röhrenförmigen Teils (108) und dem proximalen Ende (106) des zweiten röhrenförmigen Teils (102) definiert ist, wobei die Öffnung konfiguriert ist, sich an dem wenigstens einen Zweiglumen (S) auszurichten und die Fluidströmung zwischen dem wenigstens einen Zweiglumen und dem bogenförmigen Teil (L) des Lumens zu ermöglichen;
wobei der erste röhrenförmige Teil, der zweite röhrenförmige Teil und der Verbindungsteil ein einzelumflochtenes Metallrohr umfassen.

2. Medizinische Vorrichtung nach Anspruch 1, wobei die wenigstens eine Schicht aus metallischem Material mehrere Schichten aus metallischem Material umfasst.

3. Medizinische Vorrichtung nach Anspruch 1, wobei der erste und der zweite röhrenförmige Teil (108, 102) jeweils konfiguriert sind, auf einen kleineren Durchmesser beschränkt zu sein als die entsprechende erweiterte thermofixierte Konfiguration zum Abgeben in einem Katheter und Zurückkehren in die entsprechende erweitere thermofixierte Konfiguration, wenn von dem Katheter aus eingesetzt.

4. Medizinische Vorrichtung nach Anspruch 1, wobei der Verbindungsteil (114) in der Länge einstellbar ist.

5. Medizinische Vorrichtung nach Anspruch 1, wobei der erste röhrenförmige Teil (108) ein Stenttransplantat ist, das konfiguriert ist, dem bogenförmigen Teil (L) des Lumens nachgelagert angeordnet zu sein.

6. Medizinische Vorrichtung nach Anspruch 5, wobei der zweite röhrenförmige Teil (102) konfiguriert ist, die medizinische Vorrichtung dem bogenförmigen Teil des Lumens (L) vorgelagert zu verankern.

7. Medizinische Vorrichtung nach Anspruch 1, wobei eine Lage jedes des Verbindungsteils (114) und der Öffnung (116) im bogenförmigen Teil (L) des Lumens drehbar von einer Lage des wenigstens einen Zweiglumens (S) abhängig ist.

8. Medizinische Vorrichtung nach Anspruch 1, wobei der erste röhrenförmige Teil (108) konfiguriert ist, in einer Aorta thoracica descendens angeordnet zu sein, der zweite röhrenförmige Teil konfiguriert ist, in einer Aorta thoracica ascendens angeordnet zu sein, und der Verbindungsteil (114) konfiguriert ist, in einem Aortenbogen angeordnet zu sein, wobei die Öffnung (116) konfiguriert ist, sich nach der wenigstens einen sich von dem Aortenbogen aus erstreckenden Arterie auszurichten.

9. Medizinische Vorrichtung nach Anspruch 1, wobei das erste und das zweite röhrenförmige Element (108, 102) selbstausdehnbar sind.

10. Medizinische Vorrichtung nach Anspruch 1, wobei der erste und der zweite röhrenförmige Teil (108, 102) in eine bogenförmige voreingestellte Form gekrümmt sind.

11. Medizinische Vorrichtung nach Anspruch 1, wobei die Zielstelle im Lumen ein Aortenaneurysma enthält.

12. Medizinische Vorrichtung nach Anspruch 1, wobei wenigstens eine Schicht konfiguriert ist, wenigstens teilweise den Blutfluss dahindurch zu hemmen.

13. Medizinische Vorrichtung nach Anspruch 1, wobei der Verbindungsteil einen Durchmesser aufweist, der kleiner ist als ein Durchmesser jedes des ersten und des zweiten röhrenförmigen Teils, wenn der erste und der zweite röhrenförmige Teil sich in der erweiterten thermofixierten Konfiguration befinden.

14. Medizinische Vorrichtung nach Anspruch 1, wobei der Querschnitt des Verbindungsteils geringer ist als die erweiterten Querschnitte des ersten und des zweiten röhrenförmigen Teils.

## Revendications

1. Dispositif médical (100) destiné à traiter un site cible au sein d'une lumière ayant une partie arquée (L) et au moins une lumière en dérivation (S) s'étendant depuis celle-ci, ledit dispositif médical comprenant :
une première partie tubulaire (108) comprenant une extrémité proximale (112) et une extrémité distale (110) ;
une seconde partie tubulaire (102) comprenant une extrémité proximale (106) et une extrémité distale (104), les première et seconde parties tubulaires comprenant chacune au moins une couche de matériau métallique en alliage à mémoire de forme, fixé par la chaleur en une configuration fixée par la chaleur dilatée ;
une partie de liaison élastique (114) couplant ladite extrémité distale de la première partie tubulaire et l'extrémité proximale de la seconde partie tubulaire et comprenant une configuration arquée, mémorisée et préfixée configurée pour se conformer à au moins une partie de la partie arquée (L) de la lumière opposée à la au moins une lumière de dérivation (S), la partie de liaison étant configurée pour adopter sa configuration arquée mémorisée et préfixée lorsqu'elle est déployée au niveau du site cible, la partie de liaison comprenant un alliage à mémoire de forme métallique et ayant été contrainte durant une opération de fixation par la chaleur de façon à être fixée par la chaleur dans une configuration allongée axialement pour réduire son diamètre et pour mémoriser la configuration à diamètre réduit allongée axialement ; et
une ouverture (116) définie entre l'extrémité distale (110) de ladite première partie tubulaire (108) et l'extrémité proximale (106) de ladite seconde partie tubulaire (102), ladite ouverture étant configurée pour s'aligner avec la au moins une lumière de dérivation (S) et faciliter un flux fluidique entre la au moins une lumière de dérivation et la partie arquée (L) de la lumière ;
dans lequel ladite première partie tubulaire, ladite seconde partie tubulaire et ladite partie de liaison comprennent un tube métallique tressé unique.

2. Dispositif médical selon la revendication 1, dans lequel ladite au moins une couche de matériau métallique comprend une pluralité de couches de matériau métallique.

3. Dispositif médical selon la revendication 1, dans lequel lesdites première et seconde parties tubulaires (108, 102) sont configurées chacune pour être contraintes à un diamètre plus petit que la configuration fixée par la chaleur dilatée respective pour une administration au sein d'un cathéter et un retour à la configuration fixée par la chaleur dilatée respective lorsqu'elles sont déployées depuis le cathéter.

4. Dispositif médical selon la revendication 1, dans lequel ladite partie de liaison (114) est réglable en longueur.

5. Dispositif médical selon la revendication 1, dans lequel ladite première partie tubulaire (108) est une greffe d'endoprothèse configurée pour être positionnée en aval de la partie arquée (L) de la lumière.

6. Dispositif médical selon la revendication 5, dans lequel ladite seconde partie tubulaire (102) est configurée pour ancrer le dispositif médical en amont de la partie arquée de la lumière (L).

7. Dispositif médical selon la revendication 1, dans lequel un emplacement de chacune de ladite partie de liaison (114) et de ladite ouverture (116) au sein de la partie arquée (L) de la lumière dépend en rotation d'un emplacement de la au moins une lumière de dérivation (S).

8. Dispositif médical selon la revendication 1, dans lequel ladite première partie tubulaire (108) est configurée pour être positionnée au sein d'une aorte thoracique descendante, ladite seconde partie tubulaire est configurée pour être positionnée au sein d'une aorte thoracique ascendante, et ladite partie de liaison (114) est configurée pour être positionnée au sein d'un arc aortique, et dans lequel ladite ouverture (116) est configurée pour s'aligner avec au moins une artère s'étendant depuis l'arc aortique.

9. Dispositif médical selon la revendication 1, dans lequel lesdits premier et second éléments tubulaires (108, 102) sont autodilatables.

10. Dispositif médical selon la revendication 1, dans lequel lesdites première et seconde parties tubulaires (108, 102) sont incurvées en une forme préfixée arquée.

11. Dispositif médical selon la revendication 1, dans lequel ledit site cible au sein de la lumière comporte un anévrisme aortique.

12. Dispositif médical selon la revendication 1, dans lequel au moins une couche est configurée pour inhiber au moins partiellement le flux sanguin à travers celui-ci.

13. Dispositif médical selon la revendication 1, dans lequel la partie de liaison a un diamètre inférieur à un diamètre de chacune des première et seconde parties tubulaires lorsque les première et seconde parties tubulaires sont dans la configuration fixée par la chaleur dilatée.

14. Dispositif médical selon la revendication 1, dans lequel la section en coupe de la partie de liaison est inférieure aux sections en coupe dilatées des première et seconde parties tubulaires.
